# EUROPÄISCHE PATENTSCHRIFT

(11) **EP 0 790 064 B1**
(45) Veröffentlichungstag und Bekanntmachung des Hinweises auf die Patenterteilung: **18.07.2001**
(21) Anmeldenummer: 97101853.6
(22) Anmeldetag: 06.02.1997
(51) Int. Cl.: A61M 5/14, F16K 7/17

(54) **Verfahren und Infusionsbesteck zum aufeinanderfolgenden Entleeren von mehreren, insbesondere zwei Behältern mit Flüssigmedikamenten**
Procedure and infusion set for consecutively emptying a plurality,in particular two recipients containing liquid drugs
Procédé et jeu de perfusion pour vider consécutivement plusieurs, en particulier eux recipients contenant des médicaments liquides

(30) Priorität: 13.02.1996 DE 19605217
(43) Veröffentlichungstag der Anmeldung: 20.08.1997
(73) Patentinhaber: Filtertek B.V., County of Limerick (IE)
(72) Erfinder: Myers, Jan Willem Marinus, Ing., 5913 TP Venlo (NL)
(74) Vertreter: Brose, D. Karl, Dipl.-Ing.

(56) Entgegenhaltungen:
- US-A- 4 237 880
- US-A- 4 534 764

## Beschreibung

Die Erfindung betrifft ein Infusionsbesteck zum aufeinanderfolgenden Entleeren von mehreren, insbesondere von zwei, mit Flüssigmedikamenten gefüllten Behältern innerhalb des Infusionsbesteckes, bei dem das Flüssigmedikament über Zuleitungen, ein Ventil und eine Tropfkammer durch eine Rollenklemme gesteuert zum vorderen Ende des Infusionsbestecks geleitet wird, (siehe zum Beispiel US-A-4 237 880)

Zwischen den Infusionsbehältern und der Tropfkammer ist im allgemeinen ein Ventil angeordnet, das der Mengenregulierung dient. Diese Funktion wird bei bekannten Ventilen durch Ausbildung als Rückschlagventil ergänzt. Derartige Rückschlagventile weisen eine Membranscheibe auf (DE 40 39 814 A1; DE 43 04 949 A1). Je nach Druckbeaufschlagung auf der Vorder- oder Rückseite der Membranscheibe wird dabei ein Durchlaßweg freigemacht oder versperrt.

Im vorliegenden Fall besteht das Problem, daß nach Leerlaufen eines Infusionsbehälters ein gefüllter neuer Behälter an die Stelle des entleerten Behälters gebracht werden muß, was üblicherweise mit mehreren Manipulationen verbunden ist.

Der Erfindung liegt die Aufgabe zugrunde, bei Vorhandensein von zumindest zwei Behältern ein möglichst unklompliziertes und schnelles Umschalten von einem ersten auf einen zweiten Behälter zu ermöglichen.

Die gestellte Aufgabe wird erfindungsgemäß dadurch gelöst, daß das Flüssigmedikament aus nebeneinander aufgehängten Behältern derart über ein Differenzdruckventil in die Tropfkammer geleitet wird, daß während des Beginns durch den höheren Flüssigkeitsdruck und einer größeren Differenzkraftfläche des ersten Behälters vor einer Membranscheibe der Flüssigkeitsstrom aus dem zweiten Behälter gestoppt bleibt und daß der Flüssigkeitsstrom des weitestgehend leergelaufenen ersten Behälters bei niedrigerem Flüssigkeitsdruck und kleinerer Differenzkraftfläche des ersten Behälters vor der Membranscheibe auf den zweiten Behälter automatisch umgeschaltet wird. Dadurch wird neben dem automatischen Umschalten ein Entleeren des ersten Behälters ermöglicht, ohne Luftblasen den Zutritt zu gestatten.

Die Erfindung betrifft das Infusionsbesteck mit Behältern für Flüssigmedikamente, die über Zuleitungen und jeweils ein Ventil an eine Tropfkammer angeschlossen und über eine weitere Zuleitung bis zum vorderen Ende mittels einer Rollenklemme gesteuert geführt sind.

Das Umschaltelement zum Öffnen eines gefüllten Behälters, nachdem ein anderer Behälter entleert wurde, ist erfindungsgemäß dahingehend gestaltet, daß das Ventil aus einem Differenzdruckventil besteht, in das jeweils eine Eingangs-Zuleitung von einem Flüssigmedikament-Behälter in eine pro Flüssigmedikament-Behälter vorgesehene Differenzkraft-Kammer geführt ist und daß jeweils zwei Differenzkraft-Kammern mittels einer Membranscheibe voneinander abdichtbar getrennt sind, wobei beide Differenzkraft-Kammern zusammen mit einer Ableitung für das Flüssigmedikament verbunden sind. Dadurch wird das Umschalten von einer Infusionslösung auf eine gleiche andere oder auf zwei unterschiedliche Infusionslösungen automatisch ermöglicht.

Nach einer Ausgestaltung ist vorgesehen, daß das Ventil aus zwei dichtend gegeneinanderlegbaren Ventilgehäusehälften gebildet ist, wobei eine Ventilgehäusehälfte einen Ableitungs-Eingang und jede Ventilgehäusehälfte einen Zuleitungs-Eingang aufweist. Der Vorteil besteht in der Zusammenführung von zwei Flüssigkeitsströmen aus den unterschiedlichen Differenzkraft-Kammern.

Eine Verbesserung der Erfindung besteht ferner darin, daß die beiden gegeneinanderlegbaren Ventilgehäusehälften innerhalb der Differenzkraft-Kammern jeweils konzentrisch zu einem Flüssigkeitskanal ringförmige Stege aufweisen, wobei dem ersten Flüssigmedikament-Behälter ein Ringsteg mit größerem Durchmesser und dem zweiten Flüssigmedikament-Behälter ein Ringsteg mit kleinerem Durchmesser zugeordnet ist. Durch diese Ausbildung ist nur eine Membranscheibe erforderlich, die durch die Ringstege gestützt am Umfang der beiden Ventilgehäusehälften gleichmäßig gespannt angeordnet sein kann und daher die wichtige Funktion des Dichtens sicher übernimmt.

Die vorstehende Überlegung führt dazu, daß die Membranscheibe mit einem Teil des Umfangs der beiden gegeneinanderlegbaren Ventilgehäusehälften an einer Öffnung liegt, die in die Ableitung mündet. Demzufolge wird für die beiden Flüssigkeitsströme eine gemeinsame Ableitung gebildet.

Diese und weitere Funktionen werden vorteilhafterweise dadurch erfüllt, daß die koaxial zu den Ringstegen mündenden Flüssigkeitskanäle jeweils abgewinkelt geformt sind.

Die Erfindung wird sodann derart weiterentwickelt, daß an die abgewinkelt geformten Flüssigkeitskanäle jeweils Entlüftungsleitungen angeschlossen sind, in denen hydrophobische Filtermembranen eingesetzt sind. Der Vorteil besteht in der Entlüftungsmöglichkeit der Eingangs-Zuleitungen, der Differenzkraft-Kammern und der Ableitung.

Die angestrebte Dichtwirkung bei sehr kleinen Drücken wird dadurch erzielt, daß die kreisrunde Membranscheibe aus einer Flüssig-Silikon-, Silikon- oder Naturgummi-Bahn oder einer Flüssig-Silikon-, Silikon- oder Naturgummi-Matte hergestellt ist.

Ein Ausführungsbeispiel der Erfindung ist in der Zeichnung dargestellt und wird im folgenden näher beschrieben.

Es zeigen
- Fig. 1: eine Gesamtansicht des Infusionsbestecks,
- Fig. 2: eine Vorderansicht auf das Differenzdruckventil,
- Fig. 3: einen axialen Mittenschnitt durch das Differenzdruckventil mit Entlüftungsleitungen gemäß Fig. 2,
- Fig. 4: eine Vorderansicht auf das Differenzdruckventil ohne Entlüftungsleitungen und
- Fig. 5: einen axialen Mittenschnitt durch das Differenzdruckventil gemäß Fig. 4.

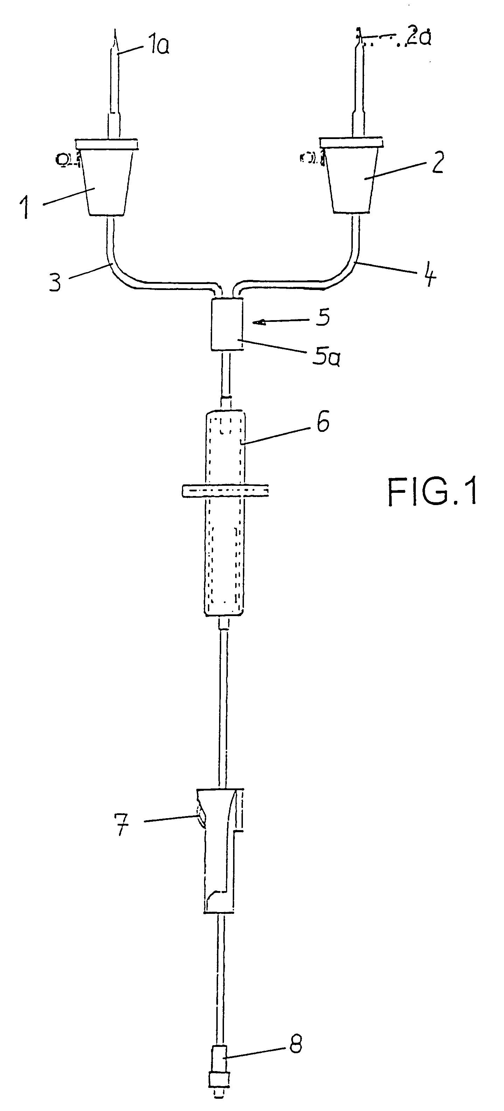

Gemäß Fig. 1 wird ein erster Behälter 1 für eine ausgewählte Medikamentflüssigkeit eingesetzt, die sich von derjenigen in einem zweiten Behälter 2 unterscheiden kann oder gleich ist. Das Flüssigmedikament wird durch Einstechen mittels der Dorne 1a bzw. 2a z.B. aus einer Ampulle eingebracht. Der erste Behälter 1 ist über eine Eingangs-Zuleitung 3 und der zweite Behälter 2 über eine Eingangs-Zuleitung 4 an ein Ventil 5 angeschlossen, das aus einem noch näher zu beschreibenden Differenzdruckventil 5a besteht. Das Flüssigmedikament gelangt durch das Ventil 5 hindurch in eine Tropfkammer 6 und wird gesteuert durch eine Rollenklemme 7 bis an das vordere Ende 8 des Infusionsbestecks geleitet, wo es wie üblich in den Körper eines Patienten gelangt.

Die Funktionsweise wird nachstehend erklärt:

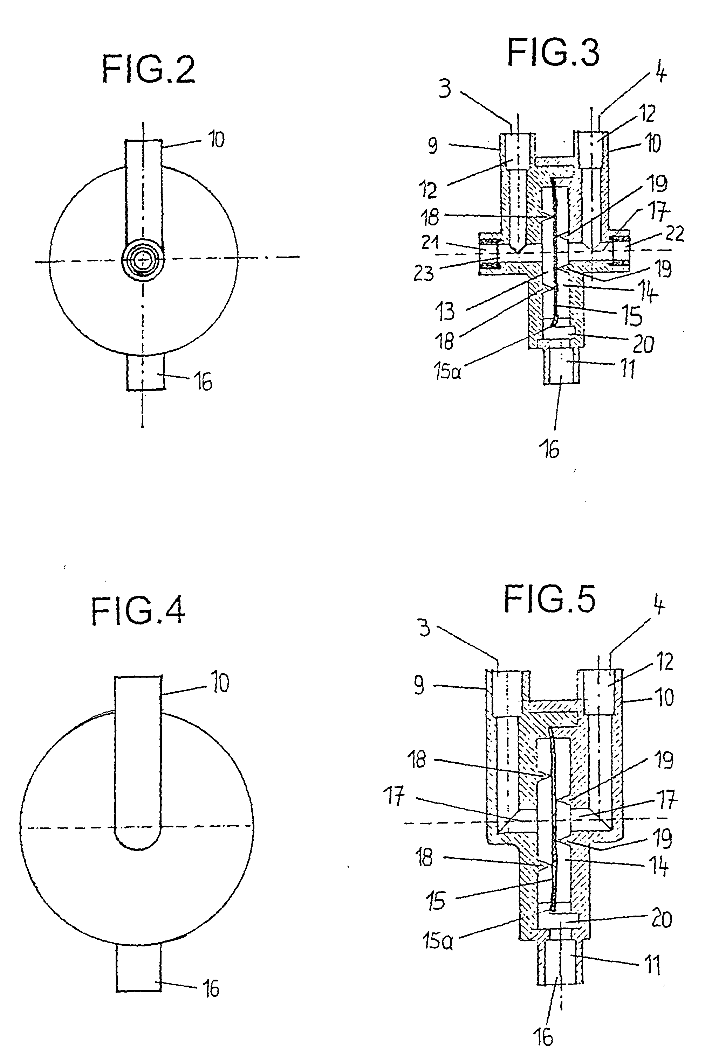

Das Flüssigmedikament wird aus den nebeneinander aufgehängten Behältern 1 oder 2 über das Differenzdruckventil 5 in die Tropfkammer 6 geleitet, wobei während des Beginns der Flüssigkeitsströmung aus dem ersten Behälter 1 durch höheren Flüssigkeitsdruck und einer größeren Differenzkraftfläche des ersten Behälters 1 vor einer Membranscheibe 15 (Fig. 3 und 5) der Flüssigkeitsstrom aus dem zweiten Behälter 2 gestoppt bleibt, da die Membranscheibe 15 wegen der größeren Differenzkraftfläche den Zustrom von Flüssigmedikament aus dem zweiten Behälter 2 unterbindet. Sobald der Flüssigkeitsstrom des ersten Behälters 1, wenn dieser weitestgehend leergelaufen ist, abebbt, sinkt der Flüssigkeitsdruck, so daß die größere Differenzdruckfläche auf der vorderen Seite der Membranscheibe 15 unter die durch die hohe Flüssigkeitssäule des zweiten Behälters 2 erzeugte Öffnungskraft fällt, wodurch automatisch vom ersten Behälter 1 auf den zweiten Behälter 2 umgeschaltet wird.

In das Differenzdruckventil 5a führt eine Eingangs-Zuleitung 3 des ersten Behälters 1 und eine Eingangs-Zuleitung 4 des zweiten Behälters 2. Beide Zuleitungen 3 und 4 münden in einer Differenzkraft-Kammer 13 bzw. 14. Die beiden Differenzkraft-Kammern 13 und 14 sind mittels der Membranscheibe 15 voneinander dichtend abtrennbar, wobei beide Differenzkraft-Kammern 13 und 14 zusammen über eine Ableitung 16 für das Flüssigmedikament verbunden sind.

Das Ventil 5 bzw. das Differenzdruckventil 5a ist aus zwei dichtend gegeneinanderlegbaren Ventilgehäusehälften 9 und 10 zusammengesetzt, und nach Montage der Membranscheibe 15 werden die Ventilgehäusehälften 9 und 10 miteinander dicht verschweißt. Eine der Ventilgehäusehälften 9 oder 10 besitzt einen Ableitungs-Eingang 11 für den Anschluß einer Zuleitung, und jede Ventilgehäusehälfte 9 und 10 weist einen Zuleitungs-Eingang 12 auf.

Die beiden gegeneinanderlegbaren Ventilgehäusehälften 9 und 10 sind innerhalb der Differenzkraft-Kammern 13 und 14 zusammen mit der Membranscheibe 15 durch ringförmige Stege 18 bzw. 19 begrenzt. Dem ersten Flüssigmedikament-Behälter 1 ist ein Ringsteg 18 mit größerem Durchmesser und dem zweiten Flüssigmedikament-Behälter 2 ein Ringsteg 19 mit kleinerem Durchmesser zugeordnet.

Wie in den Fig. 3 und 5 gezeigt ist, liegt die Membranscheibe 15 mit einem Teil des Umfangs 15a der beiden gegeneinanderlegbaren Ventilgehäusehälften 9 und 10 an einer Öffnung 20, die in die Ableitung 16 mündet.

Die koaxial zu den Ringstegen 18 bzw. 19 mündenden Flüssigkeitskanäle 17 sind abgewinkelt geformt.

An die abgewinkelt geformten Flüssigkeitskanäle 17 sind jeweils Entlüftungsleitungen 21 bzw. 22 angeschlossen (Fig. 3), wobei alternativ auf solche Entlüftungsleitungen im Hinblick auf anderweitig vorgesehene Entlüftungsmaßnahmen verzichtet werden kann (Fig. 5). In den Entlüftungsleitungen 21 bzw. 22 sind jeweils hydrophobische Filtermembranen 23 eingesetzt, die zwar luftdurchlässig, jedoch nicht für Flüssigkeiten durchlässig sind.

Die kreisrunde Membranscheibe 15 ist aus einer Flüssig-Silikon-, Silikon- oder Naturgummi-Bahn oder einer Flüssig-Silikon-, Silikon- oder Naturgummi-Matte hergestellt.

Der Einsatz des beschriebenen Infusionsbesteckes erfolgt z.B. bei Einsatz von zwei hintereinander auf den Patienten zu übertragenden Infusionslösungen. Während des Leerlaufens des ersten Behälters 1 saugt die Flüssigkeitsströmung aus dem Bereich des zweiten Behälters 2 Luft an, die gegebenenfalls entlüftet wird. Danach wird die Rollenklemme 7 auf die entsprechende Tropfgeschwindigkeit eingeregelt und der erste Behälter 1 wird entleert, wonach die Umschaltung automatisch auf den zweiten Behälter 2 erfolgt. Während dieser Zeit wird durch den Überdruck der Flüssigkeitsströmung aus dem zweiten Behälter 2 die Membranscheibe 15 gegen den Ringsteg 18 gedrückt, dagegen vom Ringsteg 19 abgehoben, so daß die Medikamentflüssigkeit aus dem zweiten Behälter 2 abströmen kann.

### Bezugszeichenliste:

- 1: erster Behälter
- 1a: Dorn
- 2: zweiter Behälter
- 2a: Dorn
- 3: Eingangs-Zuleitung
- 4: Eingangs-Zuleitung
- 5: Ventil
- 5a: Differenzdruckventil
- 6: Tropfkammer
- 7: Rollenklemme
- 8: vorderes Ende
- 9: Ventilgehäusehälfte
- 10: Ventilgehäusehälfte
- 11: Ableitungs-Eingang
- 12: Zuleitungs-Eingang
- 13: Differenzkraft-Kammer
- 14: Differenzkraft-Kammer
- 15: Membranscheibe
- 15a: Membranscheibenumfang
- 16: Ableitung
- 17: Flüssigkeitskanal
- 18: ringf. Steg / Ringsteg
- 19: ringf. Steg / Ringsteg
- 20: Öffnung
- 21: Entlüftungsleitung
- 22: Entlüftungsleitung
- 23: hydroph. Filtermembrane

## Patentansprüche

1. Infusionsbesteck mit Behältern (1; 2) für Flüssigmedikamente, die über Zuleitungen (3; 4) und ein Differenzdruckventil (5a) an eine Tropfkammer (6) angeschlossen und über eine weitere Zuleitung bis zum vorderen Ende (8) mittels einer Rollenklemme (7) gesteuert geführt sind, wobei in das Differenzdruckventil (5a) jeweils eine Eingangs-Zuleitung (3; 4) von einem Flüssigmedikament-Behälter (1; 2) in eine pro Flüssigmedikament-Behälter (1; 2) vorgesehene Differenzkraft-Kammer (13; 14) geführt ist, und jeweils zwei Differenzkraft-Kammern (13; 14) mittels einer Membranscheibe (15) voneinander abdichtbar getrennt sind, **dadurch gekennzeichnet,** daß beide Differenzkraft-Kammern (13; 14) zusammen mit einer Ableitung (16) für das Flüssigmedikament verbunden sind.

2. Infusionsbesteck nach Anspruch 1, dadurch gekennzeichnet, daß das Ventil (5) aus zwei dichtend gegeneinanderlegbaren Ventilgehäusehälften (9; 10) gebildet ist, wobei eine Ventilgehäusehälfte (9; 10) einen Ableitungs-Eingang (11) und jede Ventilgehäusehälfte (9; 10) einen Zuleitungs-Eingang (12) aufweist.

3. Infusionsbesteck nach einem der Ansprüche 1 oder 2, dadurch gekennzeichnet, daß die beiden gegeneinanderlegbaren Ventilgehäusehälften (9; 10) innerhalb der Differenzkraft-Kammern (13; 14) jeweils konzentrisch zu einem Flüssigkeitskanal (17) ringförmige Stege (18; 19) aufweisen, wobei dem ersten Flüssigmedikament-Behälter (1) ein Ringsteg (18) mit größerem Durchmesser und dem zweiten Flüssigmedikament-Behälter (2) ein Ringsteg (19) mit kleinerem Durchmesser zugeordnet ist.

4. Infusionsbesteck nach einem der Ansprüche 1 bis 3, dadurch gekennzeichnet, daß die Membranscheibe (15) mit einem Teil des Umfangs (15a) der beiden gegeneinanderlegbaren Ventilgehäusehälften (9; 10) an einer Öffnung (20) liegt, die in die Ableitung (16) mündet.

5. Infusionsbesteck nach einem der Ansprüche 1 bis 4, dadurch gekennzeichnet, daß die koaxial zu den Ringstegen (18, 19) mündenden Flüssigkeitskanäle (17) jeweils abgewinkelt geformt sind.

6. Infusionsbesteck nach einem der Ansprüche 1 bis 5, dadurch gekennzeichnet, daß an die abgewinkelt geformten Flüssigkeitskanäle (17) jeweils Entlüftungsleitungen (21; 22) angeschlossen sind, in denen hydrophobische Filtermembranen (23) eingesetzt sind.

7. Infusionsbesteck nach einem der Ansprüche 1 bis 6, dadurch gekennzeichnet, daß die kreisrunde Membranscheibe (15) aus einer Flüssig-Silikon-, Silikon- oder Naturgummi-Bahn oder einer Flüssig-Silikon-, Silikon- oder Naturgummi-Matte hergestellt ist.

## Claims

1. Infusion set having containers (1; 2) for liquid medicines, which by feedlines (3; 4) and a differential pressure valve (5a) are connected to a drip chamber (6) and which by means of a further feedline controlled by a roller clamp (7) are leading to a front end (8), wherein into the differential pressure valve (5a) each an entry feedline (3; 4) from one liquid medicine container (1; 2) is leading into a differential force chamber (13; 14), one of which each being provided for each liquid medicine container (1; 2) and, wherein each two differential force chambers (13; 14) by means of a diaphragm disk (15) sealably are separated from each other, characterized in that both differential force chambers (13; 14) together are connected to a drainline (16) for the liquid medicine.

2. Infusion set according to claim 1, characterized in that the valve (5) is formed by two valve housing halves (9; 10) sealingly contactable with each other, wherein one valve housing half (19) is having a drainline entry (11) and, wherein each valve housing half (9; 10) is having a feedline entry (12).

3. Infusion set according to any of the claims 1 or 2, characterized in that the two contactable valve housing halves (9; 10) within the differential force chambers (13; 14) are having annular ridges (18; 19), which each are concentric to a fluid channel (17), wherein an annular ridge (18) having a larger diameter is related to the first fluid medicine container (1) and an annular ridge (19) with a smaller diameter is related to the second fluid medicine container (2).

4. Infusion set according to any of the claims 1 to 3, characterized in that the diaphragm disk (15) with a part of the circumference (15a) of the two contactable valve housing halves (9; 10) is positioned at an opening (20), which is leading into the drainline (16).

5. Infusion set according to any of the claims 1 to 4, characterized in that the liquid channels (17) opening concentrically are each having an angular shape.

6. Infusion set according to any of the claims 1 to 5, characterized in that the angularly shaped fluid channels (17) each are connected to venting lines (21; 22) into which hydrophobic filter diaphragms (23) are inserted.

7. Infusion set according to any of the claims 1 to 6, characterized in that the circular diaphragm disk (15) is produced from a sheet of liquid silicon, silicon or natural rubber or from a mat of liquid silicon, silicon or natural rubber.

## Revendications

1. Trousse d'infusion comportant des récipients (1, 2) pour des médicaments liquides, qui sont alimentés par des lignes d'alimentation (3, 4) et une soupape à pression differentielle (5a) à une chambre de goutte-à-goutte (6) et sont guidés de façon commandée jusqu'à l'extrémité avant (8), au moyen d'un élément de serrage à galet (7), par une autre conduite d'alimentation, une conduite d'alimentation d'entrée (3 ; 4) respective reliant un récipient à médicament liquide (1 ; 2) à une chambre à force différentielle (13 ; 14) prévue pour chaque récipient à médicament liquide (1 ; 2) étant reliée à la soupape à pression différentielle (5a) et chaque fois, deux chambres à force différentielle (13 ; 14) étant séparées de façon à pouvoir être rendues étanches l'une de l'autre, au moyen d'un disque-membrane (15), caractérisée en ce que les deux chambres à force différentielle (13 ; 14) sont reliées ensemble à une conduite d'évacuation (16) destinée au médicament liquide.

2. Trousse d'infusion selon la revendication 1, caractérisée en ce que la soupape (5) est constituée de deux demi-carters de soupape (9 ; 10) pouvant être placés l'un contre l'autre de façon étanche, un demi-carter de soupape (9 ; 10) présentant une entrée de dérivation (11) et chaque demi-carter de soupape (9 ; 10) présentant une entrée de conduite d'alimentation (12).

3. Trousse d'infusion selon l'une des revendications 1 ou 2, caractérisée en ce que les deux demi-carters de soupape (9 ; 10) pouvant être placés l'un contre l'autre présentent, à l'intérieur de chaque chambre à force différentielle (13 ; 14), des nervures (18 ; 19) à forme annulaire, concentriques par rapport à un canal à liquide (17), une nervure annulaire (18) à plus grand diamètre étant associée au premier récipient à médicament liquide (1) et une nervure annulaire (19) à plus petit diamètre étant associée au deuxième récipient à médicament liquide (2).

4. Trousse d'infusion selon l'une des revendications 1 à 3, caractérisée en ce que le disque-membrane (15) est situé, par une partie de la périphérique (15a) des deux demi-carters de soupape (9 ; 10) pouvant être placés l'un contre l'autre, sur une ouverture (20) qui débouche dans la conduite d'évacuation (16).

5. Trousse d'infusion selon l'une des revendications 1 à 4, caractérisée en ce que les canaux à liquide (17) débouchant coaxialement par rapport aux nervures annulaires (18, 19) ont chacun une forme coudée.

6. Trousse d'infusion selon l'une des revendications 1 à 5, caractérisée en ce que à chacun des canaux à liquide (17) à forme coudée sont raccordées des conduites de désaération (21 ; 22) dans lesquelles sont insérées des membranes filtrantes (23) hydrophobes.

7. Trousse d'infusion selon l'une des revendications 1 à 6, caractérisée en ce que le disque-membrane (15), qui est circulaire, est fabriqué à partir d'une bande de silicone liquide, de silicone ou de caoutchouc naturel, ou bien d'un matelas à base de silicone liquide, de silicone ou de caoutchouc naturel.
